# EUROPEAN PATENT APPLICATION

(11) **EP 1 709 980 A1**
(43) Date of publication of application: **11.10.2006**
(21) Application number: 05007344.4
(22) Date of filing: 05.04.2005
(51) Int. Cl.: A61L 9/03, A01M 1/20

(54) **Emanation device**

(71) Applicant: Reckitt Benckiser (UK) Limited, Slough, Berkshire SL1 3UH (GB)
(72) Inventor: The designation of the inventor has not yet been filed
(74) Representative: Instone, Terry

(57) **Abstract**

An air treatment system comprises a device which can receive a tablet 10 comprising a sublimable carrier substance and an active agent. The device includes a heater 6 to cause the tablet to be given up to the air. A thermal barrier 16 located between the heater and the tablet prolongs the life of the tablet.

## Description

The present invention relates to an emanation device and to an emanation method.

Electrical devices are known for vaporising an active agent, such as an insecticide, from a solid article, in the form of a tablet. A device of this type is described in WO 97/45008. Over an extended period the active agent is vaporised into the air, leaving the solid article exhausted of active agent. The solid article is removed and discarded, and a replacement solid article is inserted in its place. This type of device is particularly popular in hot countries and/or countries which are prone to infestation by insects such as mosquitoes. However it is not without problems. Firstly, it is not possible to tell whether a solid article in place in the device is exhausted of active agent, or has been inserted recently - perhaps by another member of the household. Secondly, there is some inconvenience in having to remove and discard the exhausted solid article, insert the replacement solid article and discard the exhausted solid article. There is even a risk of becoming confused in undertaking this task and re-inserting an exhausted solid article, especially if there are a number of such devices in the property.

In WO 2004/037964 there is described a textile treatment delivery system adapted to impart a textile conditioning composition and a fragrance to a fabric while it is being dried in a heated dryer. The delivery system comprises at least one textile conditioning composition and at least one fragrance in a sublimable carrier substance.

In US 5,647,052 there is described a volatile substance dispenser which provides an indicator of dissipation of a quantity of volatile substance. Volatile substance is provided in a holding means or tray. A heat source is provided. The indicator may be a light bulb.

Problems with the arrangement described in US 5,647,052 are that providing an electrical end of life indicator is complicated and expensive, and that if it fails the device must be scrapped, unless it is designed to be replaceable - itself introducing further complication and expense.

It is an object of embodiments of the present invention to provide a simple, robust, inexpensive emanation device with end of life indication.

In accordance with a first aspect of the present invention there is provided an emanation device comprising:
a solid article comprising an active agent, wherein the solid article substantially disappears, by being given up to the air over an interval, when heated;
a site at which the solid article is retained;
a heater adjacent to the site;
and a thermal barrier between the heater and the site whereby delivery of heat to a solid article at the site is reduced;
wherein the solid article is visible at the site and wherein its substantial disappearance is the sole indicator that a replacement solid article is required.

The interval suitably represents the period of emanation, and is longer than would have been the case if the thermal barrier were not present.

When we state that the solid article substantially disappears over an interval when heated, we mean that it disappears to a sufficient extent that any solid residue therefrom is insignificant (for example it may fall or be blown away from the site). If there are any residues they do not inhibit the insertion of a replacement tablet, for example require any cleaning equipment.

Preferably the solid article is in the form of a tablet, bar, disc, sheet, plaque or the like. Most preferably it is a tablet.

By solid article, it is meant that the article is in the form of an elastic or plastic solid or a granular compressed solid at conventional consumer product storage temperatures of 37°C or less.

Preferably the solid article comprises a sublimable solid, as a carrier substance.

Preferably the active agent when incorporated into the tablet is a sublimable solid or, most preferably, a vaporisable liquid. The active agent emanated into the air is preferably in the form of a fine droplet dispersion or in a gaseous form. The active agent may exhibit its activity in the air - for example as a fragrance - or on settlement onto a surface - for example a surface sanitising agent.

By sublimable, it is meant that a substance undergoes a phase conversion from solid to vapour phase without passing through a liquid phase. The sublimation temperature is defined here as the temperature at which the vapour in equilibrium with the solid has a vapour pressure of one standard atmosphere (101.325kPa).

By vaporisable, it is meant that a substance undergoes a phase conversion from liquid to vapour phase.

The solid article may be described as evanescent, in that, whatever the mechanism may be, it substantially disappears over an interval.

In practice, a substance will sublime, or vaporise, at a temperature below the sublimation, or vaporisation, temperature provided that the ambient vapour pressure of the substance in the surrounding atmosphere is less than the equilibrium vapour pressure. For the practical applicability of the compositions according to the invention, the sublimation, or vaporisation, temperature of the sublimable, or vaporisable, material should be such that at a temperature of 60°C, the equilibrium vapour pressure is 0.1 standard atmospheres or more, preferably 0.3 or more, more preferably 0.5 or more.

The sublimation, or vaporisation, temperature of a sublimable, or vaporisable, carrier substance is suitably from 45 to 225°C, preferably from 50 to 150°C, more preferably from 60 to 100°C.

When a sublimable, or vaporisable, active agent is present, its sublimation, or vaporisation, temperature is likewise suitably from 45 to 225°C, preferably from 50 to 150°C, more preferably from 60 to 100°C.

A particularly preferred sublimable carrier substance is adamantane. Adamantane is tricyclo (3,3,1,1) decane and has been found to give particularly good sublimation behaviour in combination with low environmental impact and toxicity.

As mentioned above the active agent may be vaporisable. Thus it may be a liquid adsorbed onto or absorbed into a sublimable carrier substance.

Preferably the active agent is selected from an insecticide, an insect repellent, a miticide, an allergen deactivant, and a sanitising agent.

When an insecticide is present as an active agent, it may be selected from the insecticide classes: carbamates (for example bendiocarb and methomyl); nicotinyls (for example imidacloprid); organophosphates (for example malathion, parathion, chlorpyrifos and diazinon) and, most preferably, pyrethroids (for example allethrin, pyrethrum, phenothrin, fenvalerate, bifenthrin, cyfluthrin, etofenprox, lambda cyhalothrin, prallethrin, deltamethrin, esfenvalerate, fenrpropathrin, flucythrinate, fluvalinate, tefluthrin, tralomethrin, acinathrin, imiprothrin, esbiothrin, cyhalothrin, permethrin and cypermethrin).

A synergist may be present to boost the insecticidal activity, for example piperonyl butoxide, in the case of the pyrethroid insecticides.

When an insect repellent is present as an active agent it is preferably selected from permethrin, ethyl hexandiol and, especially, N,N-diethyl-meta-toluamide (DEET).

When a miticide is present as an active agent it is preferably selected from bifenazate, permethrin, extoxazole, spirodiclofen, milbemectin, pyridaben, fenpyroximate and acequinocyl.

When an allergen deactivant is present as an active agent it may suitably be a terpene hydrocarbon. Preferably it is selected from an essential oil (for example a citrus oil, especially orange oil), L-ascorbic acid, tea tree oil, tannic acid, thymol and glutaraldehyde.

When a sanitizing agent is present as an active agent it is preferably selected from pyrithiones (especially zinc pyrithione, also known as ZPT); dimethyldimethylol hydantoin (Glydant);
methylchloroisothiazolinone/methylisothiazolinone (Kathon CG); imidazolidinyl urea (Germall 115) and diazolidinyl urea (Germall II); glutaraldehyde; halogenated diphenyl ethers, for example 2,4,4-trichloro-2-hydroxy-diphenyl ether (Triclosan or TCS), and 2,2-dihydroxy-5,5-dibromo-diphenyl ether; phenolic compounds, for example alkylphenols and halophenols (and including bisphenols and resorcinols); benzoic esters (parabens), for example methylparaben, propylparaben, butylparaben, ethylparaben, isopropylparaben, isobutylparaben, benzylparaben, sodium methylparaben, and sodium propylparaben; halogenated carbanilides (e.g., 3,4,4-trichlorocarbanilides (Triclocarban or TCC); and, especially, germicidal quaternary ammonium compounds such as cetyl trimethyl ammonium bromide, octadecyl dimethyl benzyl ammonium bromide, N-cetyl pyridinium bromide, octyl phenoxy ethoxy ethyl dimethyl benzyl ammonium chloride and N-(laurylcocoaminoformylmethyl)-pyridinium chloride, lauryloxyphenyltrimethyl ammonium chloride, cetylaminophenyltrimethyl ammonium methosulphate, dodecylphenyltrimethyl ammonium methosulphate dodecylphenyltrimethyl ammonium chloride and chlorinated dodecylphenyltrimethyl ammonium chloride.

Preferred quaternary ammonium compounds which act as sanitising agents have the structural formula: 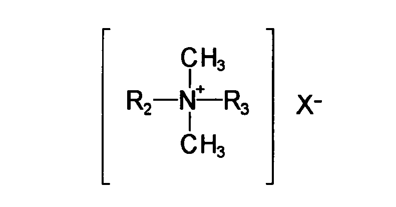 wherein R₂ and R₃ are the same or different C₈-C₁₂ alkyl, or R₂ is C₁₂-C₁₆ alkyl, C₈-C₁₈ alkylethoxy, C₈-C₁₈ alkyl-phenylethoxy and R₂ is benzyl, and X is a halide ion, for example chloride, bromide or iodide, or a methosulphate ion. The alkyl groups R₂ and R₃ may be straight chain or branched, but are preferably substantially linear.

The solid article may contain a fragrance. All known fragrances may be considered for use in the present invention: a skilled formulator would have no difficulty in the selection of a suitable fragrance or fragrance blend.

There may be more than one sublimable carrier substance present in the solid article.

There may be more than one active agent present in the solid article. For example the solid article may contain two insecticides, with different spectra of insecticidal behaviour. Another example of a solid article may contain an insecticide and an insect repellent. Another example may contain a miticide, an allergen deactivant and a sanitising agent.

Preferably the sublimable carrier substance constitutes at least 90% w/w of the solid article, preferably at least 95% w/w, and more preferably at least 98% w/w. Preferably the carrier substance comprises up to 99.99% w/w of the solid article, preferably up to 99.9% w/w, and more preferably up to 99.6% w/w. When there is more than one sublimable or carrier substance present the figures given above apply to the total complement of such substances. The active agent preferably comprises up to 10% w/w of the solid article, preferably up to 5% w/w, more preferably up to 2% w/w. Preferably the active agent comprises at least 0.01% w/w of the solid article, preferably at least 0.1% w/w, and more preferably at least 0.4% w/w. When there is more than one active agent present - for example an insecticide and a sanitising agent - the figures given above apply to the total complement of such materials.

The solid article may contain a substance or substances which are neither sublimable or vaporisable. Such substances may have a vapour pressure of not more than 100 Pa at 20°C; and preferably not more than 200 Pa at 20°C. However as noted above the solid article should substantially disappear to the extent that a new solid article can readily be inserted once the previous solid article has substantially disappeared. In practice this generally means that such materials should not be present in an amount exceeding 0.5 wt%, preferably not exceed 0.3 wt%, most preferably not exceeding 0.2 wt%. Also, the residue from such materials is preferably discontinuous in nature - for example powdery.

The solid article could contain further components. For example it may contain a component to reduce brittleness. Alternatively or additionally it may contain a binder or plasticiser or mould release aid, for example magnesium stearate. However any further component should still permit the solid article to be substantially given up to the air.

Preferably the solid article is in the form of a body having two major side walls. Preferably those major side walls are substantially flat (or only deviate mildly from that condition). Preferably, however, those major side walls are substantially parallel to each other. Preferably each major side wall is of area at least 2 cm², preferably at least 4 cm². Preferably each major side wall is of area not exceeding 10 cm², more preferably not exceeding 6 cm². Preferably the solid article is thin. Preferably its thickness does not exceed 3 mm. More preferably it does not exceed 2.5 mm, and most preferably it does not exceed 2 mm. For reasons of structural integrity it is preferably of thickness at least 1 mm, more preferably at least 1.4 mm, and most preferably at least 1.6 mm.

Preferably the solid article weighs at least 0.2g, preferably at least 0.4g.

Preferably the solid article weighs not more than 2g, preferably not more than 1.2g, more preferably not more than 1g, and most preferably not more than 0.8g.

Preferably the relative density of the solid article is at least 0.4, more preferably at least 0.5.

Preferably the relative density of the solid article is not more than 0.9, more preferably not more than 0.75.

It will be appreciated that all of the physical definitions given above for the solid article refer to the solid article before any diminution has taken place due to sublimation and/or vaporisation.

Preferably the solid article is supplied in an individual, substantially airtight, package. Preferably a plurality of solid articles is provided, hermetically separated from each other but in common packaging. Examples of suitable packaging include aluminium or plastics flow wrapping, and blister packs.

The solid article could be manufactured by any available manufacturing method, but is preferably formed by compression moulding of a particulate feedstock, for example using a rotary press.

The heater is preferably in the form of a hotplate. Preferably it has a flat surface.

Preferably the site for retention of the solid article comprises means for holding it at the site. The site may therefore comprise a housing or mounting for the solid article. Preferably the means for holding comprises a resilient member which is displaced on introduction of the solid article to the site, and thereby produces a holding force. Preferably a holding force is still provided as the solid article diminishes in size, in use. Preferably a holding force is provided substantially until the solid article loses its structural integrity or is given up in its entirety to the air. Suitably the resilient means comprise at least one flexible part (for example flexible fin(s) or tang(s) or spring(s)) which is/are deflected by the solid article in a direction of its insertion into the device. Preferably the resilient means, in the rest position, terminate only slightly short of a surface which the solid article will abut, in use.

The resilient means could be carried by the thermal barrier or heater but preferably are carried by the casing, and suitably project therefrom towards the thermal barrier and heater.

The purpose of the thermal barrier is to increase the working life of the solid article. It does this by reducing delivery of heat to the site, and consequently to the solid article. Preferably the thermal barrier entirely screens the site for the solid article, from the heater. Thus its major dimensions, length and width, are preferably equal to or greater than the major dimensions of the solid article.

The thermal barrier may suitably be a body secured over the heater, as a part of the device. "Naked" solid articles are then located at the site. Alternatively the thermal barrier may be a body moved into place at the site, for use. For example it may be a body conjoined to a solid article and the solid article and thermal barrier together inserted into place. It may be fixedly conjoined, in the sense that the solid article could not be separated from the thermal barrier, or the solid article and the thermal barrier could be brought together for insertion. Each solid article may have its own thermal barrier. Preferably, however, with a plurality of solid articles (for example a pack) a smaller number of thermal barriers is provided and most preferably only one thermal barrier per pack of replacement solid articles. When the thermal barrier is a body which can be moved into place at the site it may be provided with one or more tabs or other holding aids, preferably projecting from the thermal barrier in a direction in alignment with the direction in which the thermal barrier is moved into or removed from the site.

The thermal barrier is preferably a body which is self-supporting in form. It may conform to the shape of the solid article. It may be of a material with a low thermal conductivity, for example of glass (especially glass fibres), ceramic, graphite, or a polymeric material, for example aliphatic or aromatic polyamides, e.g. Nylon or Kevlar (Trade Marks), suitably in the form of a fabric, or PTFE and silicones, suitably in the form of a non-fibrous mat. 1-5 layers of material in a ply may suitably be used. Suitable ceramics include fibre mats of aluminoborosilicate or aluminosilicate or alumina fibres.

The thermal barrier could in principle be a body which is itself consumed during operation, albeit more slowly than the solid article. Preferably, however, it is not consumed, but is not degraded or caused to change state by the heat it is subjected to, in use.

The thermal barrier may be a body which is heat-reflective, for example mirrored, at least on a surface facing the heater.

The thermal barrier may be a body which is solid, in the sense of not being space-enclosing in nature.

Alternatively or additionally the thermal barrier may be a body which is space-enclosing in nature. For example it may be corrugated, or fluted, or cellular, or fibrous. If fibrous, it may be of a non-woven or a woven material. It may be of a metallic material, preferably in the form of a fibrous or cellular form; an example is aluminium mesh.

The thermal barrier may be a body in the form of a plate, or sheet, or shim, or card, located between the heater and the solid article, in use. In this embodiment there is preferably no air gap between the heater and the solid article.

Preferably the mean thickness of the thermal barrier in the form of a body as described in the preceding paragraphs is at least 0.3 mm, more preferably at least 0.5 mm, and most preferably at least 0.7 mm. Preferably the mean thickness of the thermal barrier does not exceed 2 mm, and preferably does not exceed 1 mm.

A thermal barrier in the form of a body as described in the preceding paragraphs preferably has a generally curved or rounded perimeter, in plan view. When of a fibrous form the edge or edges are preferably mechanically rolled over and/or heat sealed.

The thermal barrier may be constituted by a coating provided on, preferably adhered to the solid article, which prolongs the life of the solid article. Preferably such a coating comprises an inorganic particulate material. Suitable materials may include SiO₂, Al₂O₃, TiO₂, SiC, WC, ZnS, talcs, clays, ZnO or ZrO₂, phosphates and silicates, including aluminosilicates, borosilicates and phosphosilicates. Such materials may be deposited on the tablets from a liquid medium in which they are dispersed or dissolved, by immersion or by a spraying method, for example plasma spraying. Preferably the particles have a mean particle size of at least 0.1 µm, more preferably at least 1 µm, most preferably at least 5 µm. Preferably the particles have a mean particle size of up to 100 µm, more preferably up to 75 µm, most preferably up to 40 µm. Preferably the average thickness of the coating after drying is at least 1 µm, more preferably at least 5 µm. Preferably the average thickness of the coating after drying is preferably up to 200 µm, more preferably up to 100 µm, most preferably up to 50 µm. The size of the particles may be measured using a Coulter counter, calibrated to U S Sedimentometer. The size is the average of the particles across the size distribution, taken as the 50% cumulative point of the distribution curve.

In another embodiment the thermal barrier may be an air gap between the solid article and the heater. For example the solid article may be held spaced from the heater. In one embodiment the solid article may have a concave face which faces the heater. In other embodiments the heater has a concave face which faces the solid article. In such embodiments the concavity constitutes the thermal barrier. The edge or edges of the solid article may rest on the heater or may be protected and/or spaced from the heater by a shield.

The thermal barrier may be made up of both a body and an air gap. For example the body could be a part with a concave face, facing the solid article. When the body is in the form of a coating of particulates there may inevitably be an air gap arising from the discontinuous nature of the coating.

Preferably the temperature of the surface of the heater when said thermal barrier is absent is in the range 100-200°C, more preferably 110-150°C.

Preferably the temperature of the exposed surface of the thermal barrier (facing away from the heater) is in the range 40-100°C, preferably 50-90°C, especially 60-80°C.

Preferably the thermal barrier conveys heat to the solid article and the temperature of the surface of the thermal barrier (in the form of a body) which conveys heat to the solid article is at least 25°C less than the corresponding surface of the heater in the absence of the solid article; preferably at least 40°C less, and most preferably at least at least 50°C less.

Preferably the thermal barrier at least doubles the time it takes for the solid article to be given up in its entirety to the air.

Preferably the solid article takes at least one hour to be given up in its entirety to the air; more preferably at least four hours; and most preferably at least eight hours. In an especially preferred embodiment it takes at least twelve hours to be given up in its entirety to the air.

Preferably the emanation device is powered by electricity. This may be supplied by a solar cell or by one or more batteries. Preferably, however, it is supplied by mains electricity.

Preferably the solid article is visible in the emanation device without the user having to move any part. The user can see when it has disappeared, and needs to be replaced. To this end the solid article and the heater or thermal barrier beneath it may visually contrast with each other. For example one may be white and the other may be black or a colour, preferably a strong colour, such as red, blue or green. Alternatively or additionally the heater or thermal barrier may carry a notice that a new solid article is required, for example a picture or word(s), such as REPLACE.

The emanation device may have a cover through which the solid article is visible. The cover may have an opening or series of openings through which the solid article is visible. The cover may be in the form of a grille. The cover may have a slot at one side through which the solid article may be conveyed to the site.

The emanation device has no means other than the presence or absence of the solid article, to denote its status, for example no light, alarm or other electrical indicator.

In accordance with a further aspect of the present invention there is provided a refill pack for an emanation device of the first aspect, comprising one or more thermal barriers and a larger number of solid articles.

In accordance with a further aspect of the present invention there is provided a kit comprising an emanation device of the first aspect, together with further solid articles.

Preferably a plurality of solid articles is supplied in packaging which comprises respective individual, substantially airtight locations.

In accordance with a further aspect of the present invention there is provided an emanation device comprising:
a site for retention of a substantially evanescent solid article such that it is externally visible and when it has been substantially given up to the air its absence is externally visible;
a heater adjacent to the site;
and a thermal barrier between the heater and the site whereby delivery of the heat to the site is reduced;
wherein the emanation device has no indicator of the status of the solid article other than its visible presence or absence.

In accordance with a further aspect of the present invention there is provided a method of delivering an active agent into the air using an emanation device of any aspect of the present invention as defined above, the method comprising:
locating a said solid article at the site in the device;
operating the heater until the solid article has been substantially given up to the air leaving the site substantially empty; and
locating a further solid article at the site.

The invention will now be further described, by way of example, with reference to the accompanying drawings in which:
Fig. 1 shows an air treatment device in a perspective frontal view, assembled;
Fig. 2 shows the device of Fig. 1 in frontal perspective exploded view; and
Fig. 3 shows the device of Fig. 1 in a cross-sectional view in the plane indicated as A-A¹, in Fig. 1.

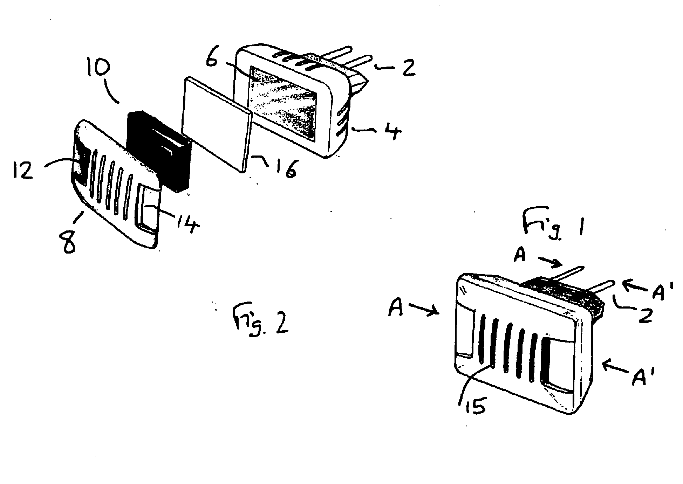

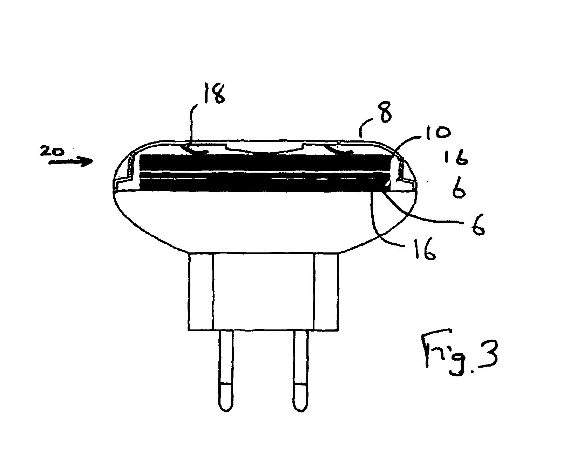

The device shown in Figs. 1 to 3 is a plug-in electrical device for repelling and killing mosquitoes. It has electrical pins 2, a heater 4 in the form of a hotplate 6, and a cover grille 8 to clip or otherwise be secured, over the hot plate. Between the hotplate 6 and the cover grille 8 there is a site for location of a solid article, namely a mosquito killing tablet 10 in this embodiment.

The cover grille 8 has a side opening 12, 14 on each of its sides, by means of which the naked tablet 10 can be located in place under the cover grille, without the need to remove or displace the cover grille itself. Between the side openings it has an array of grille slits 15. The tablet is coloured and can easily be seen, through the slits.

A thermal barrier in the form of an insulating pad 16 is located on the hotplate. When a tablet is located in place it exactly overlies the insulating pad. Thus, the tablet is entirely screened from the hotplate 6 by the insulating pad 16. The function of the insulating pad is to slow down the delivery of heat to the tablet, and prolong its working life.

The tablet is rectangular, of size 2.94 cm x 1.69 cm x 0.175 cm, and of relative density of 0.69. It weighs 0.6g when introduced into the device. The insulating pad is of a triple ply of soft ceramic fabric/aluminium mesh/soft ceramic fabric, of mean thickness 0.75 m. Each ceramic fabric is of aluminosilicate fibres, available as Nextel 720 (Trade Mark).

Fig. 3 shows clearly the arrangement of hotplate 6, insulating pad 16 and tablet 10; with the insulating pad 16 sandwiched between the hotplate 6 and the tablet 10. It can also be seen that the cover grille 8 has, on its inner surface, two fins 18. Each fin is of a flexible elastomeric nature and is co-moulded with the cover grille. The fins are shown displaced in one direction. This is the direction in which the tablet was moved to insert it into the apparatus. The fins were displaced by the tablet when it was inserted. The direction of insertion is shown by arrow 20. The fins, in seeking to return to their rest position, provide a force which acts upon the tablet, urging it to remain in position.

The tablet is made up of adamantane (98.7% w/w), and insecticides (d-allethrin, 1.1%; d-phenothrin, 0.2% w/w). The adamantane is impregnated with the other components, thoroughly mixed, and then pressed into shape. The tablets may be packaged in blister packs comprising a plastics tray sealed over with foil. The tablets may be sealed individually in the pockets of the blister pack and may be removed one at a time. Replacement blister packs will be available to consumers. In the case of large-scale manufacture it may be desired to replace 0.1 %w/w of the adamantane with magnesium stearate, as a lubricant or pressing aid. However this may not be necessary, especially if the tabletting equipment has PTFE-coated punches and dies.

Over an extended period the solid article is given up into the atmosphere, and substantially disappears, or evanesces. The adamantane sublimes and the insecticides are volatile liquids, which evaporate. If magnesium stearate is present it is not sublimable but disappears, either being carried away with the other materials as they sublime or evaporate, or forming a scarcely perceptible residue which may readily be dispersed e.g. by blowing it away.

In an experiment using a commercially available plug-in fragrancing device sold under the Trade Mark Mortein Rodasol, and without the Nextel 720/aluminium mesh/Nextel 720 insulating pad described above, a tablet as described above introduced into the device was given up entirely within 30 minutes. When the same device was modified so that the inserted tablet and the hotplate were separated by the insulating pad, this period may be extended to 8 hours, or more.

In other embodiments the working life of the tablet could be extended by means of different types of insulating pad. Examples of other suitable materials are fibreglass (with or without resin binder), aluminium mesh, aromatic polyamide fabric, graphite sheet, PTFE sheet, ceramic fabric, cork granules bound with synthetic rubber binder, and board material. A single layer may be used, or a plurality of layers.

Users can readily tell when a replacement tablet is needed: the previous solid article is no longer present, and so can no longer be seen, through the slits 15 in the cover grille 8. A new solid article can then be inserted in its place, without any residue having to be removed first.

In this embodiment the insulating pad may be secured in place over the hotplate. In another embodiment the insulating pad is in the form of a shim or tray which is insertable into and removable from the device. The insulating pad and solid article may be brought together and inserted into the device. When the tablet has substantially disappeared the insulating pad may be removed and the new solid article and insulating pad combination inserted into the device. One packet of tablets may be supplied with one insulating pad.

In the embodiments having a non-fixed insulating pad, to assist insertion and removal the insulating pad may have two tabs, projecting from the insulating pad in opposed directions in alignment with the direction in which the insulating pad is moved into place, one at each end.

Alternatively each solid article may be supplied with its own insulating pad, preferably being secured thereto.

Alternatively the base of each solid article maybe coated with a heat retarding coating, for example of inorganic particulate material.

## Claims

1. An emanation device comprising:
a solid article comprising an active agent, wherein the solid article substantially disappears by being given up to the air, over an interval, when heated;
a site at which the solid article is retained;
a heater adjacent to the site;
and a thermal barrier between the heater and the site whereby delivery of heat to a solid article at the site is reduced;
wherein the solid article is visible at the site and wherein its substantial disappearance is the sole indicator that a replacement solid article is required.

2. An emanation device according to claim 1 wherein the thermal barrier is a generally flat body of mean thickness in the range 0.5 mm to 2 mm.

3. An emanation device according to claim 1 or 2 wherein the solid article is a generally flat body of mean thickness in the range 1 mm to 4 mm.

4. An emanation device according to any preceding claim wherein the site for location of the solid article comprises means for retaining the solid article at the site.

5. An emanation device according to claim 4 wherein the means for retaining comprises resilient means which is displaced on introduction of the solid article to the site the displaced resilient means thereby producing a holding force on the solid article.

6. An emanation device according to claim 5 wherein the resilient means comprise at least one flexible part displaced by the solid article in a direction of its insertion into the device.

7. An emanation device according to any preceding claim wherein the solid article is visible at the site without the user having to move any part of the device.

8. An emanation device according to any preceding claim wherein the solid article comprises a sublimable carrier substance.

9. An emanation device according to claim 8 wherein the sublimable carrier substance is adamantane.

10. An emanation device according to any preceding claim wherein the solid article comprises a vaporisable or sublimable active agent selected from an insecticide, an insect repellent, a miticide, an allergen deactivant and a sanitizing agent.

11. An emanation device according to any preceding claim wherein the thermal barrier is fixed at the site as part of the device.

12. An emanation device according to any of claims 1 to 10 wherein the thermal barrier is moved into place at the site, for use.

13. An emanation device as claimed in claim 12 wherein the thermal barrier is conjoined to the solid article.

14. A refill pack for an emanation device as claimed in claim 12 or 13, comprising one or more thermal barriers and a larger number of solid articles.

15. A kit comprising an air treatment device according to any of claims 1 to 13, together with further solid articles.

16. A refill pack or kit according to claim 14 or 15, wherein the solid articles are supplied in packaging which comprises respective individual, substantially airtight, locations.

17. An emanation treatment device comprising:
a site for retention of a substantially evanescent solid article such that it is externally visible and when it has been substantially given up to the air its absence is externally visible;
a heater adjacent to the site;
and a thermal barrier between the heater and the site whereby delivery of the heat to the site is reduced;
wherein the emanation device has no indicator of the status of the solid article other than its visible presence or absence.

18. A method of delivering an active agent into the air using an emanation device according to any of claims 1 to 13 or 17, the method comprising:
locating a said solid article at the site in the device;
operating the heater until the solid article has been substantially given up to the air leaving the site substantially empty; and
locating a further solid article at the site.
